# EUROPÄISCHE PATENTSCHRIFT

(11) **EP 1 412 555 B1**
(45) Veröffentlichungstag und Bekanntmachung des Hinweises auf die Patenterteilung: **07.11.2007**
(21) Anmeldenummer: 02764600.9
(22) Anmeldetag: 27.06.2002
(51) Int. Cl.: C23F 11/14, E21B 41/02, C07C 217/08

(54) **KORROSIONSINHIBITOREN MIT VERBESSERTER WASSERLÖSLICHKEIT UND ERHÖHTER FILMPERSISTENZ**
CORROSION INHIBITORS WITH IMPROVED WATER SOLUBILITY AND IMPROVED FILM PERSISTENCE
INHIBITEURS DE CORROSION AMELIORES EN TERMES DE SOLUBILITE DANS L'EAU ET DE RESISTANCE PELLICULAIRE

(30) Priorität: 13.07.2001 DE 10134226
(43) Veröffentlichungstag der Anmeldung: 28.04.2004
(73) Patentinhaber: Clariant Produkte (Deutschland) GmbH, 65929 Frankfurt am Main (DE)
(72) Erfinder: DAHLMANN, Uwe, 69126 Heidelberg (DE); FEUSTEL, Michael, 55278 Köngernheim (DE)
(86) Internationale Anmeldenummer: PCT/EP2002/007097
(87) Internationale Veröffentlichungsnummer: WO 2003/008668

(56) Entgegenhaltungen:
- EP-A- 0 320 769
- WO-A-98/23792
- PATENT ABSTRACTS OF JAPAN vol. 006, no. 261 (C-141), 21. Dezember 1982 (1982-12-21) & JP 57 152475 A (SANYO KASEI KOGYO KK;OTHERS: 01), 20. September 1982 (1982-09-20)

## Beschreibung

Die vorliegende Erfindung betrifft ein Additiv und ein Verfahren zur Korrosionsinhibierung an Einrichtungen zur Förderung und Transport von Kohlenwasserstoffen in der Erdölförderung und -verarbeitung.

In technischen Prozessen, bei denen Metalle mit Wasser oder auch mit Öl-Wasser-Zweiphasensystemen in Kontakt kommen, besteht die Gefahr der Korrosion. Besonders in Salzwassersystemen, wie sie in Erdölgewinnungs- und Verarbeitungsprozessen vorkommen, ist diese Gefahr besonders ausgeprägt. Ohne spezielle Additive zum Schutz der eingesetzten Ausrüstungen sind die Ausbeutung einer Lagerstätte und die Verarbeitung des Erdöls nicht möglich.

Solche Korrosionsschutzmittel sind zwar schon seit langem bekannt, jedoch in vielerlei Hinsicht noch nicht optimal. Viele Produkte, z.B. Amide/Imidazoline aus Fettsäuren und Polyaminen sind zu sehr öllöslich und somit in der korrosiven Wasserphase aufgrund schlechter Verteilungsgleichgewichte (Partitioning) nur in geringer Konzentration vorhanden. Demgemäss sind diese als Korrosionsschutzmittel nur wenig oder nur bei hoher Dosierung wirksam.

DE-A-199 30 683 beschreibt Amidamine/Imidazoline, die durch Umsetzung von Alkylpolyglykolethercarbonsäuren mit Polyaminen erhalten werden, und aufgrund ihrer Struktur eine sehr gute Wasserlöslichkeit besitzen und somit durch gutes Partitioning verbesserten Korrosionsschutz besitzen.

Quartäre Alkylammoniumverbindungen (Quats) stellen alternative Korrosionsschutzmittel des Standes der Technik dar, die neben den korrosionsinhibierenden auch biostatische Eigenschaften besitzen. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Des weiteren beschränkt die schlechte biologische Abbaubarkeit ihren Einsatz in ökologisch sensiblen Anwendungsgebieten.

US-5 523 433 offenbart Verbindungen der Formel 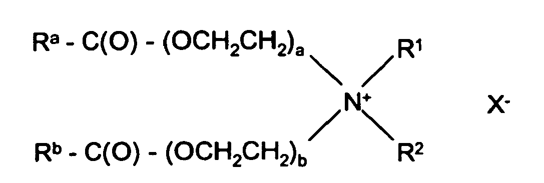 worin R^{a} und R^{b} für C₁₂- bis C₂₂-Alkylreste und R¹ und R² für C₁- bis C₄-Alkylreste stehen können. Das Dokument offenbart die Eignung solcher Verbindungen als Bestandteil von Wäscheweichspülern.

EP-B-0 736 130, EP-B-0 824 631, US-5 648 575 und WO-99/13197 offenbaren Verfahren zur Inhibierung von Gashydraten unter Verwendung von alkoxylierten Alkylammoniumverbindungen.

US-6 025 302 offenbart Polyetheraminammoniumverbindungen als Gashydratinhibitoren, deren Ammoniumstickstoffatom neben der Polyetheraminkette 3 Alkylsubstituenten trägt.

WO-00/78 706 beschreibt quartäre Ammoniumverbindungen, die jedoch keine Carbonylreste tragen. Die Verwendung als Korrosionsinhibitoren wird nicht offenbart.

Aufgabe der vorliegenden Erfindung war es also, neue Korrosionsinhibitoren zu finden, die bei konstant gutem oder verbessertem Korrosionsschutz neben einer optimierten Wasserlöslichkeit, einer schnelleren Filmbildung und somit verbesserten Filmpersistenz auch eine verbesserte biologische Abbaubarkeit im Vergleich zu den Korrosionsinhibitoren des Standes der Technik bieten.

Es wurde nun überraschend gefunden, dass zweifach N-alkoxylierte und carbonylierte Ammoniumsalze ausgezeichnete Wirkung als Korrosionsinhibitoren aufweisen, sowie verbesserte Filmpersistenz und gute biologische Abbaubarkeit zeigen.

Gegenstand der Erfindung ist somit die Verwendung von Verbindungen der Formel 1 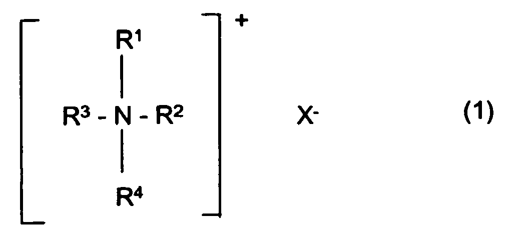 worin
- R¹, R²: einen Reste der Formel

-(A-O)ₙ-(C)-CO-O-R⁵ (3)
- R³: C₁- bis C₃₀-Alkyl oder C₂- bis C₃₀-Alkenyl
- R⁴: einen gegebenenfalls Heteroatome enthaltenden organischen Rest mit 1 bis 100 Kohlenstoffatomen
- R⁵: C₁- bis C₃₀-Alkyl oder C₂- bis C₃₀-Alkenyl
- n: eine Zahl von 1 bis 20
- A: eine C₂- bis C₄-Alkylengruppe,
- B: eine C₁- bis C₁₀-Alkylengruppe,
- C: eine C₁- bis C₆-Alkylengruppe und
- X: ein Anion
bedeuten, als Korrosionsinhibitoren.

Die Inhibitoren können in einem Verfahren zur Inhibierung von Korrosion an Metalloberflächen, insbesondere von eisenhaltigen Metallen eingesetzt werden, indem einem korrosiven System, welches mit den Metalloberflächen in Kontakt steht, mindestens eine Verbindung der Formel 1 zugesetzt wird.

Ein weiterer Gegenstand der Erfindung sind die Verbindungen der Formel (1).

Korrosive Systeme im Sinne dieser Erfindung sind bevorzugt flüssig/flüssig- bzw. flüssig/gasförmig-Mehrphasensysteme, bestehend aus Wasser und Kohlenwasserstoffen, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid. Kohlenwasserstoffe im Sinne dieser Erfindung sind organische Verbindungen, die Bestandteile des Erdöls/Erdgases sind, und deren Folgeprodukte.

A kann geradkettig oder verzweigt sein und steht vorzugsweise für eine Ethylen- oder Propylengruppe, insbesondere eine Ethylengruppe. Bei den durch (A-O)ₙ bezeichneten Alkoxygruppen kann es sich auch um gemischte Alkoxygruppen handeln.

C kann geradkettig oder verzweigt sein und steht vorzugsweise für eine C₂- bis C₄-Alkylengruppe, insbesondere für eine Methylen- oder Ethylengruppe.

n steht vorzugsweise für eine Zahl zwischen 2 und 6.

R⁵ steht vorzugsweise für eine Alkyl- oder Alkenylgruppe mit 2 bis 24 Kohlenstoffatomen, insbesondere 4 bis 12 Kohlenstoffatomen.
R³ steht vorzugsweise für eine Alkyl- oder Alkenylgruppe von 2 bis 12 Kohlenstoffatomen, insbesondere für solche Gruppen mit 4 bis 8 Kohlenstoffatomen und speziell für Butyl-Gruppen.

R⁴ kann ein beliebiger organischer Rest sein, der 1 bis 100 C-Atome enthält, und der Heteroatome enthalten kann. Enthält R⁴ Heteroatome, so handelt es sich vorzugsweise um Stickstoff- oder Sauerstoffatome oder beides, vorzugsweise um beides. Die Stickstoffatome können in quatemierter Form vorliegen.

In einer weiteren bevorzugten Ausführungsform umfasst R⁴ 1 bis 20 Alkoxygruppen, die von C₂- bis C₄-Alkylenoxid abgeleitet sind, insbesondere von Ethylenoxid und/oder Propylenoxid. Insbesondere kann R⁴ für einen Rest nach Formel (2) oder (3) stehen.

In einer besonders bevorzugten Ausführungsform entspricht R⁴ einem Rest der Formel (4) 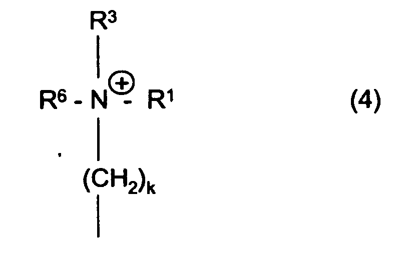 wobei die Bindung an das Stickstoffatom in Formel 1 über die freie Valenz der (CH₂)ₖ-Gruppe erfolgt. In Formel (4) bedeutet R⁶ einen Rest der Formeln

-(B)-(O-A)ₙ-O-CO-R⁵ (2)

oder

-(A-O)ₙ-(C)-CO-O-R⁵ (3)

oder C₁- bis C₃₀-Alkyl oder C₂- bis C₃₀-Alkenyl, jeweils mit den weiter oben für A, n, und R⁵ angegebenen Vorzugsbereichen. k steht für 2 oder 3, R¹ und R³ haben die oben angegebenen Bedeutungen. B kann geradkettis oder verzweigt sein und steht vorzugsweise für einen C₂-bis C₄- Alkytengruppe, insbesondere für eine Ethylen- oder Propylengruppe.

Als Gegenionen X sind alle Ionen geeignet, die die Löslichkeit der Verbindungen der Formel (1) in den korrosiven organisch-wässrigen Mischphasen nicht beeinträchtigen. Solche Gegenionen sind beispielsweise Methylsulfationen (Methosulfat) oder Halogenidionen.

Besonders bevorzugte Verbindungen (ohne Gegenionen dargestellt) entsprechen den Formeln (5) und (8)

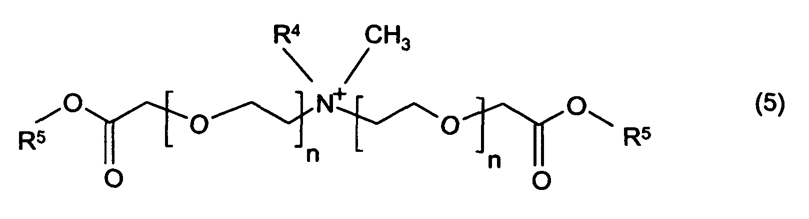

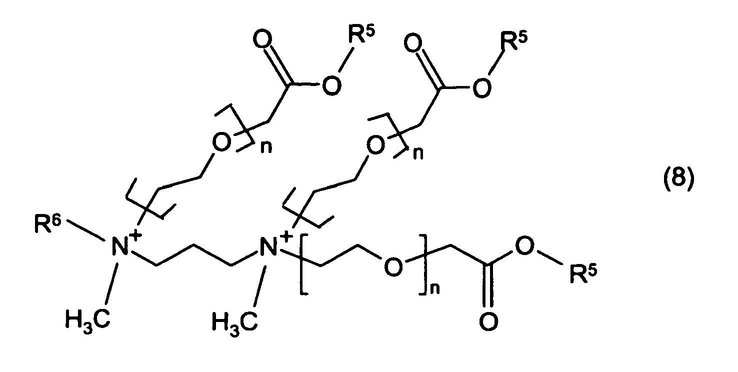

Die erfindungsgemäßen Verbindungen können alleine oder in Kombination mit anderen bekannten Korrosionsinhibitoren eingesetzt werden. Im allgemeinen wird man soviel des erfindungsgemäßen Korrosionsinhibitors einsetzen, dass man unter den gegebenen Bedingüngen einen ausreichenden Korrosionsschutz erhält. Bevorzugte Einsatzkonzentrationen bezogen auf die reinen erfindungsgemäßen Verbindungen sind 5 bis 5000 ppm, bevorzugt 10 bis 1000, insbesondere 15 bis 150 ppm.

Besonders geeignet als Korrosionsinhibitoren sind auch Mischungen der erfindungsgemäßen Produkte mit anderen literaturbekannten Korrosionsinhibitoren, wie Amidaminen und/oder Imidazolinen aus Fettsäuren und Polyaminen und deren Salzen, quartären Ammoniumsalzen, oxethylierten/oxpropylierten Aminen, Amphoglycinaten und -propionaten, Betainen oder Verbindungen beschrieben in DE-A-19 930 683.

Die erfindungsgemäßen Verbindungen können dadurch hergestellt werden, dass man alkoxylierte Alkylamine bzw. Alkylaminoalkylenamine mit Monochlorcarbonsäuren zu den entsprechenden Ethercarbonsäuren umsetzt und anschließend mit Alkanolen verestert. Andererseits können die bisalkoxylierten Monoalkylamine bzw. Alkylamino-alkylenamine direkt mit Carbonsäuren und deren Derivaten, wie Anhydride, Carbonsäurechloride bzw. deren Ester, zu den erfindungsgemäßen Estern umgesetzt werden. Danach erfolgt die Quaternierung mit geeigneten Alkylierungsmitteln.

Die Herstellung von alkoxylierten Alkylaminen bzw. Alkylaminoalkylenaminen ist im Stand der Technik beschrieben.

Basis der verwendeten alkoxylierten Alkylamine sind Alkylamine mit C₁- bis C₃₀-Alkylresten oder C₂- bis C₃₀-Alkenylresten, bevorzugt C₃- bis C₈-Alkylamine. Geeignete Alkylamine sind beispielsweise n-Butylamin, Isobutylamin, Pentylamin, Hexylamin, Octylamin, Cyclopentylamin, Cyclohexylamin.

Basis der verwendeten alkoxylierten Alkylaminoalkylenamine sind Aminoalkylenamine mit C₁- bis C₃₀-Alkylresten oder C₂- bis C₃₀-Alkenylresten und k = 2 oder 3. Geeignete Aminoalkylenamine sind beispielsweise Fettalkylpropylendiamine wie Talgfettpropylendiamin, Stearylpropylendiamin, Oleylpropylendiamin, Laurylpropylendiamin, Dodecylpropylendiamin und Octylpropylendiamin.

Die Alkylamine bzw. Alkylaminoalkylenamine werden im allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf Alkylamin bzw. Alkylaminoalkylenamine werden 1-40 Mol Alkylenoxid beaufschlagt, bevorzugt 1-12 Mol.

Die Alkoxylierung erfolgt in Substanz, kann aber auch in Lösung durchgeführt werden. Geeignete Lösemittel für die Alkoxylierung sind inerte Ether wie Dioxan, Tetrahydrofuran, Glyme, Diglyme und MPEGs.

Im Allgemeinen wird die Alkoxylierung im ersten Reaktionsschritt unkatalysiert bis auf > 95 Gew.-% tert.-Stickstoff durchgeführt. Höheralkoxylierung erfolgt nach Zugabe basischer Verbindungen als Katalysatoren. Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Für die Herstellung der erfindungsgemäßen Verbindungen werden in einem anschließenden Reaktionsschritt die Amin-Oxethylat-Mischungen mit einem Chlorcarbonsäurederivat und einer Base, bevorzugt trockenem Chloressigsäure-Natriumsalz und Natriumhydroxid umgesetzt. Dies kann geschehen, indem man die Oxethylat-Mischung mit 100 bis 150 Mol-% Natriumchloracetat bei 30 bis 100°C umsetzt und gleichzeitig oder nacheinander mit festem Natriumhydroxid oder Kaliumhydroxid versetzt, so dass die Summe aus der in der Oxethylatmischung bereits vorliegenden Base und der zusätzlich zugegebenen Basenmenge der Menge an Natriumchloracetat entspricht. Die aus der Umsetzung mit dem Alkylenoxid bereits enthaltene Basenmenge kann somit direkt für die anschließende Williamson-Synthese genutzt werden und muss nicht, wie bei der Synthese eines Standard-Oxethylats, ausgewaschen werden.

Anschließend an die Alkylierungsreaktion werden die alkoxylierten Amin-Ethercarbonsäure-Alkalisalze in die freie Ethercarbonsäure überführt. Hierzu wird mit starker Mineralsäure (Salzsäure, Schwefelsäure) auf pH < 3 angesäuert und die Ethercarbonsäure durch Phasentrennung oberhalb ihres Trübungspunktes heiß als Oberphase abgetrennt.

Die anschließende Veresterung der alkoxylierten Amin-Ethercarbonsäuren erfolgt im allgemeinen durch direkte Umsetzung der freien Säure mit entsprechenden Alkoholen bei Temperaturen von 100 - 200 °C, wobei das Reaktionswasser destillativ entfernt wird. Die Veresterung kann durch Zugabe geeigneter saurer Katalysatoren mit einem pKₐ-Wert von kleiner gleich 5 oder durch Auskreisen des Reaktionswassers mit geeigneten Lösemitteln beschleunigt werden. Geeignete Katalysatoren sind beispielsweise Sulfonsäure und Alkylstannansäuren.

Für die Veresterung der alkoxylierten Amin-Ethercarbonsäuren werden Alkohole mit C₄- bis C₃₀-Alkylresten oder C₄- bis C₃₀-Alkenylresten verwendet, bevorzugt Fettalkohole. Geeignete Alkohole stellen beispielsweise 2-Ethylhexanol, Octanol, Decanol, Laurylalkohol, Palmitylalkohol, Stearylalkohol und Oleylalkohol dar.

Die erfindungsgemäßen Verbindungen können auch durch Veresterung der Amin-Oxethylat-Mischungen mit Carbonsäuren und deren Derivaten, wie Carbonsäurechloride, Carbonsäureanhydride und Carbonsäureester hergestellt werden. Die Veresterung mit freien Carbonsäuren erfolgt bei Temperaturen von 100 - 200 °C, wobei das Reaktionswasser destillativ entfernt wird. Die Veresterung kann durch Zugabe geeigneter saurer Katalysatoren mit einem pKₐ-Wert von kleiner gleich 5 oder durch Auskreisen des Reaktionswassers mit geeigneten Lösemitteln beschleunigt werden. Geeignete Carbonsäuren stellen Essigsäure, Propionsäure, Capronsäure, Caprylsäure, 2-Ethylhexansäure und Fettsäuren bzw. deren Anhydride, Methylester und Chloride dar.

Die Herstellung der erfindungsgemäßen Verbindungen erfolgt dann durch Quaternierung der tertiären Stickstoffatome mit einem geeigneten Alkylierungsmittel bei 50 bis 150 °C. Geeignete Alkylierungsmittel stellen Alkylhalogenide und Alkylsulfate dar, bevorzugt Methylenchlorid, Butylbromid und Dimethylsulfat.

### Beispiele:

### a) Allgemeine Vorschrift für die Herstellung alkoxylierter Amin-Ethercarbonsäuren

In einer Rührapparatur wurden 2 mol des entsprechenden alkoxylierten Amins bzw. 1 mol des entsprechenden alkoxylierten Diamins (nach OH-Zahl) unter Stickstoffspülung vorgelegt und auf 40°C erwärmt. Dann wurden 650 g (4,8 mol) Natriumchloracetat für alkoxylierte Monoamine bzw. 488 g (3,6 mol) Natriumchloracetat für alkoxylierte Diamine eingetragen und die Reaktionsmischung auf 50°C erwärmt. Nach jeweils 30 min wurden 192 g (4,8 mol) bzw. 144 g (3,6 mol) NaOH-Microprills in 6 Portionen so zugegeben, dass die Temperatur 55°C nicht übersteigt. Es wurde 2 h bei 70°C nachreagiert. Danach wurde 10 % Salzsäure zudosiert, bis ein pH < 3 erreicht wurde. Die Mischung wurde dann auf 95°C erhitzt und in eine beheizbare Rührapparatur mit Bodenablass überführt. Die Phasentrennung erfolgte nach 15 min bei 105 - 108°C. Die wässrige Unterphase wurde verworfen. Bei Produkten, die sich nicht durch Erhitzen über den Trübungspunkt abtrennen lassen, wurde das Reaktionswasser destillativ entfernt und das dabei ausfallende Salz abfiltriert.

### Beispiel 1 (n-Butylamin + 6 EO-ECS)

Aus 699 g n-Butylamin + 6 EO (OH-Zahl: 321,1 mg KOH/g) wurden 970 g n-Butylamin + 6 EO-ECS mit SZ = 221,5 mg KOH/g (entspricht 91,9 % Umsatz) und bas.-N = 3,00 % erhalten.

### Beispiel 2 (Caprylamin + 6 EO-ECS)

Aus 801 g Caprylamin + 6 EO (OH-Zahl: 280,1 mg KOH/g) wurden 1045 g Caprylamin + 6 EO-ECS mit SZ = 200,9 mg KOH/g (entspricht 92,5 % Umsatz) und bas.-N = 2,69 % erhalten.

### Beispiel 3 (Caprylamin + 10 EO-ECS)

Aus 1147 g Caprylamin + 10 EO (OH-Zahl: 195,7 mg KOH/g) wurden 1412 g Caprylamin + 10 EO-ECS mit SZ = 144,9 mg KOH/g (entspricht 89,0 % Umsatz) und bas.-N = 1,90 % erhalten.

### Beispiel 4 (Talgfett-propylendiamin + 10 EO-ECS)

Aus 768 g Talgfett-propylendiamin + 10 EO (OH-Zahl: 219,2 mg KOH/g) wurden 970 g Talgfett-propylendiamin + 10 EO-ECS mit SZ = 156,7 mg KOH/g (entspricht 87,7 % Umsatz) und bas.-N = 2,88 % erhalten.

### Beispiel 5 (Talgfett-propylendiamin + 25 EO-ECS)

Aus 1316 g Talgfett-propylendiamin + 25 EO (OH-Zahl: 127,9 mg KOH/g) wurden 1700 g Talgfett-propylendiamin + 25 EO-ECS mit SZ = 85,0 mg KOH/g (entspricht 84,0 % Umsatz) und bas.-N = 1,49 % erhalten.

### Beispiel 6 (Talgfett-propylendiamin + 30 EO-ECS)

Aus 1699 g Talgfett-propylendiamin + 30 EO (OH-Zahl: 99,1 mg KOH/g) wurden 2043 g Talgfett-propylendiamin + 30 EO-ECS mit SZ = 66,5 mg KOH/g (entspricht 80,9 % Umsatz) und bas.-N = 1,30 % erhalten.

### Beispiel 7 (Talgfett-propylendiamin + 35 EO-ECS)

Aus 1919 g Talgfett-propylendiamin + 35 EO (OH-Zahl: 87,7 mg KOH/g) wurden 2301 g Talgfett-propylendiamin + 35 EO-ECS mit SZ = 63,2 mg KOH/g (entspricht 85,5 % Umsatz) und bas.-N = 1,19 % erhalten.

### Beispiel 8 (Lauryl-propylendiamin + 10 EO-ECS)

Aus 673 g Lauryl-propylendiamin + 10 EO (OH-Zahl: 250,0 mg KOH/g) wurden 1071 g Lauryl-propylendiamin + 10 EO-ECS mit SZ = 149,2 mg KOH/g (entspricht 90,5 % Umsatz) und bas.-N = 2,54 % erhalten.

### Beispiel 9 (Lauryl-propylendiamin + 30 EO-ECS)

Aus 1639 g Lauryl-propylendiamin + 30 EO (OH-Zahl: 102,7 mg KOH/g) wurden 1964 g Lauryl-propylendiamin + 30 EO-ECS mit SZ = 82,3 mg KOH/g (entspricht 97,1 % Umsatz) und bas.-N = 1,40 % erhalten.

### b) Allgemeine Vorschrift für die Herstellung alkoxylierter Amin-Ethercarbonsäurealkylester

In einer Rührapparatur wurden 1 mol bzw. 0,5 mol (nach SZ) der entsprechenden alkoxylierten Alkylamin- bzw. Alkylendiamin-Ethercarbonsäure unter Stickstoffspülung vorgelegt und mit einem Überschuss (ca. 1,5 mol Equivalente je Carbonsäurefunktion) Alkohol versetzt. Nach Zugabe von 0,5 Gew.-% FASCAT 4100 (Butylstannansäure) wurde die Mischung auf 100°C bis 180°C erhitzt, wobei das Reaktionswasser abdestillierte. Nach einer Reaktionszeit von 8 h bzw. Erreichen einer Säurezahl von SZ < 5 mg KOH/g wurde die Umsetzung beendet und überschüssiger Alkohol bzw. Restwasser destillativ im Vakuum entfernt.

### Beispiel 10 (n-Butylamin + 6 EO-2-ethylhexyl-ECS-ester)

Aus 507 g n-Butylamin + 6 EO-ECS und 391 g 2-Ethylhexanol wurden 707 g n-Butylamin + 6 EO-2-ethylhexyl-ECS-ester mit SZ = 4,1 mg KOH/g und VZ = 158,1 mg KOH/g (entspricht 97,4 % Umsatz) erhalten.

### Beispiel 11 (Caprylamin + 6 EO-2-ethylhexyl-ECS-ester)

Aus 559 g Caprylamin + 6 EO-ECS und 391 g 2-Ethylhexanol 738 g Caprylamin + 6 EO-2-ethylhexyl-ECS-ester mit SZ = 3,3 mg KOH/g und VZ = 147,0 mg KOH/g (entspricht 97,8 % Umsatz) erhalten.

### Beispiel 12 (Caprylamin + 10 EO-2-ethylhexyl-ECS-ester)

Aus 774 g Caprylamin + 10 EO-ECS und 391 g 2-Ethylhexanol wurden 999 g Caprylamin + 10 EO-2-ethylhexyl-ECS-ester mit SZ = 4,8 mg KOH/g und VZ = 114,1 mg KOH/g (entspricht 95,8 % Umsatz) erhalten.

### Beispiel 13 (Talgfett-propylendiamin + 10 EO-2-ethylhexyl-ECS-ester)

Aus 537 g Talgfett-propylendiamin + 10 EO-ECS und 293 g 2-Ethylhexanol wurden 688 g Talgfett-propylendiamin + 10 EO-2-ethylhexyl-ECS-ester mit SZ = 4,7 mg KOH/g und VZ = 121,3 mg KOH/g (entspricht 96,1 % Umsatz) erhalten.

### Beispiel 14 (Talgfett-propylendiamin + 25 EO-ethylhexyl-ECS-ester)

Aus 990 g Talgfett-propylendiamin + 25 EO-ECS und 293 g 2-Ethylhexanol wurden 1068 g Talgfett-propylendiamin + 25 EO-2-ethylhexyl-ECS-ester mit SZ = 6,7 mg KOH/g und VZ = 74,6 mg KOH/g (entspricht 91,0 % Umsatz) erhalten.

### Beispiel 15 (Talgfett-propylendiamin + 30 EO-ethylhexyl-ECS-ester)

Aus 1266 g Talgfett-propylendiamin + 30 EO-ECS und 293 g 2-Ethylhexanol wurden 1374 g Talgfett-propylendiamin + 30 EO-2-ethylhexyl-ECS-ester mit SZ = 3,5 mg KOH/g und VZ = 61,7 mg KOH/g (entspricht 94,3 % Umsatz) erhalten.

### Beispiel 16 (Talgfett-propylendiamin + 35 EO-dodecyl-ECS-ester)

Aus 1332 g Talgfett-propylendiamin + 35 EO-ECS und 419 g Laurylalkohol wurden 1523 g Talgfett-propylendiamin + 35 EO-2-dodecyl-ECS-ester mit SZ = 4,9 mg KOH/g und VZ = 54,2 mg KOH/g (entspricht 90,9 % Umsatz) erhalten.

### Beispiel 17 (Lauryl-propylendiamin + 10 EO-2-ethylhexyl-ECS-ester)

Aus 564 g Lauryl-propylendiamin + 10 EO-ECS und 293 g 2-Ethylhexanol wurden 703 g Lauryl-propylendiamin + 10 EO-2-ethylhexyl-ECS-ester mit SZ = 3,6 mg KOH/g und VZ = 117,9 mg KOH/g (entspricht 96,9 % Umsatz) erhalten.

### Beispiel 18 (Lauryl-propylendiamin + 30 EO-dodecyl-ECS-ester)

Aus 1023 g Lauryl-propylendiamin + 30 EO-ECS und 419 g Laurylalkohol wurden 1213 g Lauryl-propylendiamin + 30 EO-dodecyl-ECS-ester mit SZ = 6,0 mg KOH/g und VZ = 66,8 mg KOH/g (entspricht 91,0 % Umsatz) erhalten.

### c) Allgemeine Vorschrift für die Quaternierung der alkoxylierten Amin-Ethercarbonsäurealkylester

In einer Rührapparatur wurden 0,5 mol (nach VZ-Zahl) des entsprechenden alkoxylierten Amin- Ethercarbonsäurealkylesters unter Stickstoffspülung vorgelegt und auf 60°C erwärmt. Dazu wurden 0,4 mol Dimethylsulfat so zugetropft, dass die Reaktionstemperatur 80-90°C nicht übersteigt. Die Reaktionsmischung wurde anschließend 3 h bei 90°C nachgerührt. Nach dieser Vorschrift wurden die Verbindungen, beschrieben durch die Beispiele 10 bis 30 quatemisiert (Beispiele 31 bis 51, wie in Tabelle 1 und 2 aufgeführt).

### Wirksamkeit der erfindungsgemäßen Verbindungen als Korrosionsinhibitoren

Die erfindungsgemäßen Verbindungen wurden als Korrosionsinhibitoren im Shell-wheel-test geprüft. Coupons aus C-Stahl (DIN 1.1203 mit 15 cm² Oberfläche) wurden in eine Salzwasser/Petroleum-Mischung (9:1,5 %ige NaCl- Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich" ein Rückstandsamin - Quat auf Basis Dicocosalkyl-dimethylammoniumchlorid (Korrosionsinhibitor des Standes der Technik).

**Tabelle 1: (SHELL-Wheel-Test)**

| Beispiel | Korrosionsinhibitor | ø Schutz % |
|---|---|---|
| Vergleich | | 36,0 |
| 19 | Quat aus Beispiel 10 | 86,0 |
| 20 | Quat aus Beispiel 11 | 88,6 |
| 21 | Quat aus Beispiel 12 | 79,2 |
| 22 | Quat aus Beispiel 13 | 65,3 |
| 23 | Quat aus Beispiel 14 | 51,8 |
| 24 | Quat aus Beispiel 15 | 47,7 |
| 25 | Quat aus Beispiel 16 | 76,3 |
| 26 | Quat aus Beispiel 17 | 64,0 |
| 27 | Quat aus Beispiel 18 | 81,9 |

Die Produkte wurden außerdem im LPR- Test (Testbedingungen analog ASTM D 2776) geprüft.

**Tabelle 2: (LPR- Test)**

| Beispiel | Korrosionsinhibitor | Schutz nach [%] | | |
|---|---|---|---|---|
| | | 10 min | 30 min | 60 min |
| Vergleich | | 53,9 | 61,2 | 73,7 |
| 28 | Beispiel 31 | 74,3 | 84,8 | 87,0 |
| 29 | Beispiel 32 | 78,4 | 86,1 | 92,3 |
| 30 | Beispiel 33 | 70,2 | 74,7 | 81,0 |
| 31 | Beispiel 37 | 51,9 | 65,6 | 74,9 |
| 32 | Beispiel 39 | 53,5 | 65,9 | 75,2 |

Wie aus den obigen Testresultaten zu erkennen ist, weisen die erfindungsgemäßen Produkte sehr gute Korrosionsschutzeigenschaften bei niedriger Dosierung auf. Die Verbindungen sind biologisch abbaubar, wie im folgenden gezeigt wird

**Tabelle 3: (Sturm - Test nach OECD 301 B)**

| Beispiel | Korrosionsinhibitor | Biologischer Abbau in % |
|---|---|---|
| Vergleich | | 28 |
| 33 | Beispiel 32 | 46 |

## Patentansprüche

1. Verwendung von Verbindungen der Formel 1 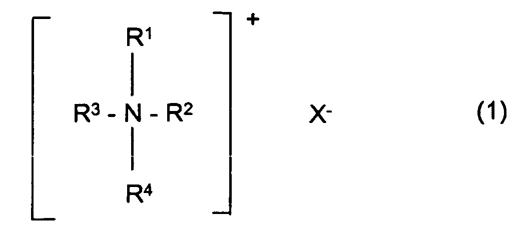 worin
R¹, R² einen Rest der Formel
-(A-O)ₙ-(C)-CO-O-R⁵ (3)
R³ C₁- bis C₃₀-Alkyl oder C₂- bis C₃₀-Alkenyl
R⁴ einen gegebenenfalls Heteroatome enthaltenden organischen Rest mit 1 bis 100 Kohlenstoffatomen
R⁵ C₁- bis C₃₀-Alkyl oder C₂- bis C₃₀-Alkenyl
n eine Zahl von 1 bis 20
A eine C₂- bis C₄-Alkylengruppe,
C eine C₁- bis C₆-Alkylengruppe und
X ein Anion
bedeuten, als Korrosionsinhibitoren.

2. Verwendung gemäß Anspruch 1, worin A für eine Ethylen- oder Propylengruppe steht.

3. Verwendung gemäß Anspruch 1 und/oder 2, worin C für eine C₂- bis C₄-Alkylengruppe steht.

4. Verwendung gemäß einem oder mehreren der Ansprüche 1 bis 3, worin n für eine Zahl zwischen 2 und 6 steht.

5. Verwendung gemäß einem oder mehreren der Ansprüche 1 bis 4, worin R⁵ für eine Alkyl- oder Alkenylgruppe mit 2 bis 24 Kohlenstoffatomen steht.

6. Verwendung gemäß einem oder mehreren der Ansprüche 1 bis 5 worin R³ für eine Alkyl- oder Alkenylgruppe von 2 bis 12 Kohlenstoffatomen steht.

7. Verwendung gemäß einem oder mehreren der Ansprüche 1 bis 6, worin R⁴ einem Rest der Formel (4) entspricht, 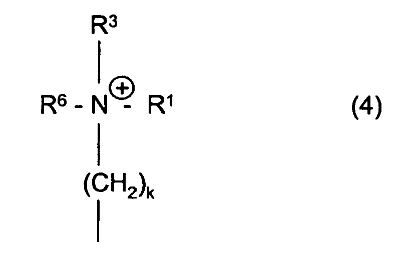 in dem R⁶ einen Rest der Formel
-(A-O)ₙ-(C)-CO-O-R⁵ (3)
oder C₁- bis C₃₀-Alkyl oder C₂- bis C₃₀-Alkenyl und k 2 oder 3 bedeutet.

8. Verwendung gemäß einem oder mehreren der Ansprüche 1 bis 7, worin Verbindungen der Formeln (5) oder (8) 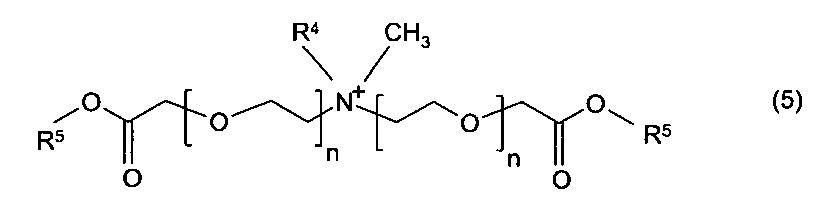 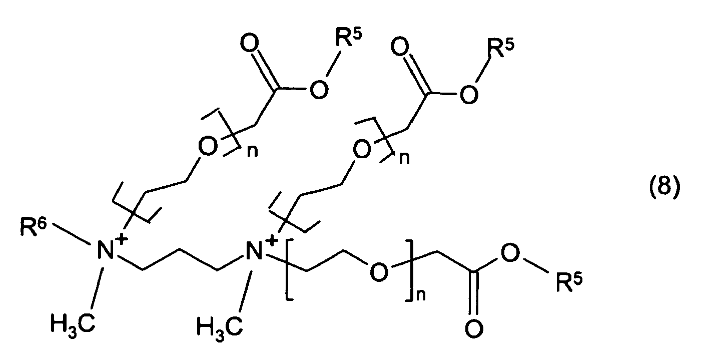 zur Anwendung kommen.

9. Verbindungen der Formel 1 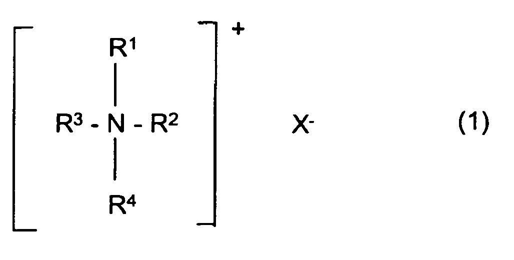 worin
R¹, R² einen Rest der Formel
-(A-O)ₙ-(C)-CO-O-R⁵ (3)
R³ C₁- bis C₃₀-Alkyl oder C₂- bis C₃₀-Alkenyl
R⁴ einen gegebenenfalls Heteroatome enthaltenden organischen Rest mit 1 bis 100 Kohlenstoffatomen
R⁵ C₁- bis C₃₀-Alkyl oder C₂- bis C₃₀-Alkenyl
n eine Zahl von 1 bis 20
A eine C₂- bis C₄-Alkylengruppe,
C eine C₁- bis C₆-Alkylengruppe und
X ein Anion
bedeuten.

## Claims

1. The use of compounds of the formula 1 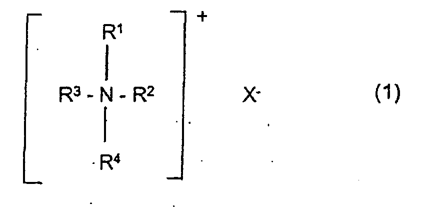 in which
R¹, R², is a radical of the formula
-(A-O)ₙ-(C)-CO-O-R⁵ (3)
R³ is C₁- to C₃₀-alkyl or C₂- to C₃₀-alkenyl
R⁴ is an organic radical having 1 to 100 carbon atoms which optionally contains heteroatoms
R⁵ is C₁- to C₃₀-alkyl or C₂- to C₃₀-alkenyl
n is a number from 1 to 20
A is a C₂- to C₄-alkylene group,
C is a C₁- to C₆-alkylene group and
X is an anion
as corrosion inhibitors.

2. The use as claimed in claim 1, in which A is an ethylene or propylene group.

3. The use as claimed in claim 1 and/or 2, in which C is a C₂- to C₄-alkylene group.

4. The use as claimed in one or more of claims 1 to 3, in which n is a number between 2 and 6.

5. The use as claimed in one or more of claims 1 to 4, in which R⁵ is an alkyl or alkenyl group having 2 to 24 carbon atoms.

6. The use as claimed in one or more of claims 1 to 5, in which R³ is an alkyl or alkenyl group having 2 to 12 carbon atoms.

7. The use as claimed in one or more of claims 1 to 6, in which R⁴ corresponds to a radical of the formula (4) 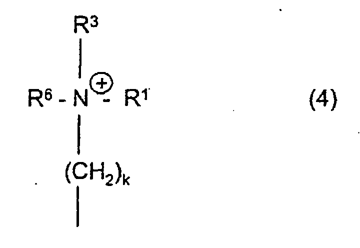 in which R⁶ is a radical of the formula
-(A-O)ₙ-(C)-CO-O-R⁵ (3)
or C₁- to C₃₀-alkyl or C₂- to C₃₀-alkenyl and k is 2 or 3.

8. The use as claimed in one or more of claims 1 to 7, in which compounds of the formulae (5) or (8) 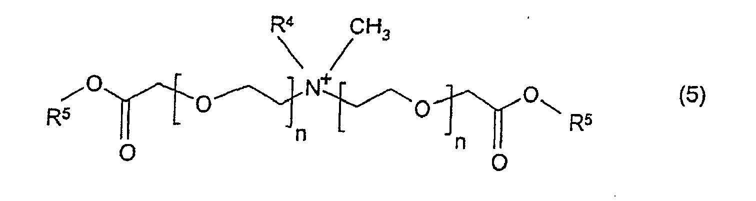 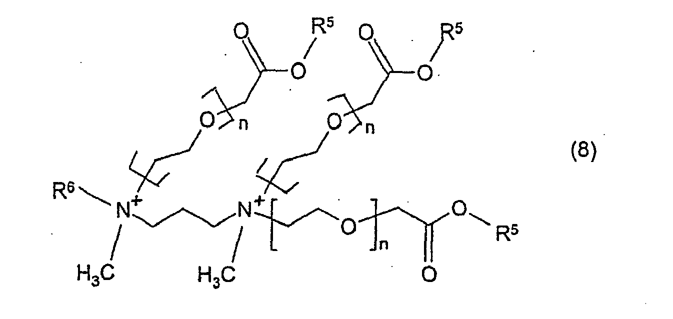 are used.

9. A compound of the formula 1 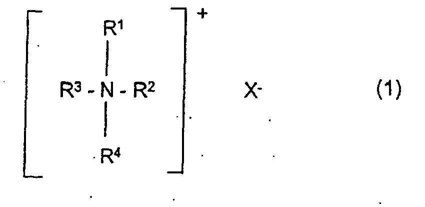 in which
R¹ R², is a radical of the formula
-(A-O)ₙ-(C)-CO-O-R⁵ (3)
R³ is C₁- to C₃₀-alkyl or C₂- to C₃₀-alkenyl
R⁴ is an organic radical having 1 to 100 carbon atoms which optionally contains heteroatoms
R⁵ is C₁- to C₃₀-alkyl or C₂- to C₃₀-alkenyl
n is a number from 1 to 20
A is a C₂- to C₄-alkylene group,
C is a C₁- to C₆-alkylene group and
X is an anion.

## Revendications

1. Utilisation de composés de formule 1 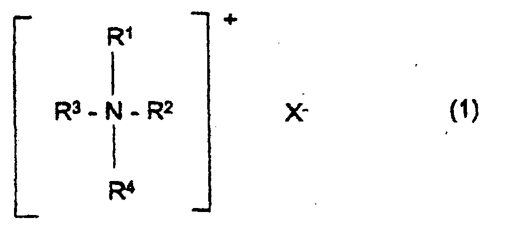 dans laquelle
R¹, R² représentent un radical de formule
-(A-O)ₙ-(C)-CO-O-R⁵ (3)
R³ un groupe alkyle en C₁ à C₃₀ ou alcényle en C₂ à C₃₀
R⁴ un radical organique, contenant le cas échéant des hétéroatomes, comportant de 1 à 100 atomes de carbone
R⁵ un groupe alkyle en C₁ à C₃₀ ou alcényle en C₂ à C₃₀
n un nombre de 1 à 20
A un groupe alkylène en C₂ à C₄
C un groupe alkylène en C₁ à C₆
X un anion,
en tant qu'inhibiteurs de la corrosion.

2. Utilisation selon la revendication 1,
dans laquelle A représente un groupe éthylène ou propylène.

3. Utilisation selon la revendication 1 et/ou la revendication 2,
dans laquelle C représente un groupe alkylène en C₂ à C₄.

4. Utilisation selon l'une quelconque ou plusieurs des revendications 1 à 3,
dans laquelle n représente un nombre compris entre 2 et 6.

5. Utilisation selon l'une quelconque ou plusieurs des revendications 1 à 4,
dans laquelle R⁵ représente un groupe alkyle ou alcényle comportant de 2 à 24 atomes de carbone.

6. Utilisation selon l'une quelconque ou plusieurs des revendications 1 à 5,
dans laquelle R³ représente un groupe alkyle ou alcényle de 2 à 12 atomes de carbone.

7. Utilisation selon l'une quelconque ou plusieurs des revendications 1 à 6,
dans laquelle R⁴ correspond à un radical de formule (4) 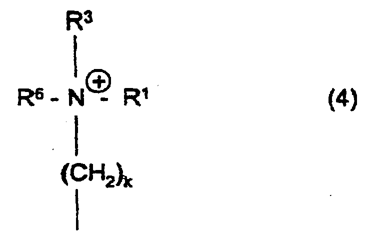 dans laquelle R⁶ représente un radical de formule
- (A-O)ₙ-(C)-CO-O-R⁵ (3)
ou un groupe alkyle en C₁ à C₃₀ ou alcényle en C₂ à C₃₀ et k est égal à 2 ou 3.

8. Utilisation selon l'une quelconque ou plusieurs des revendications 1 à 7,
dans laquelle on utilise des composés des formules (5) ou (8)

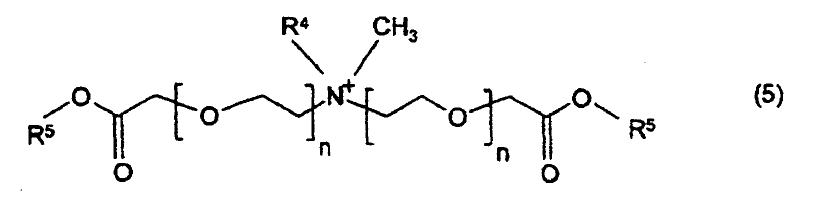

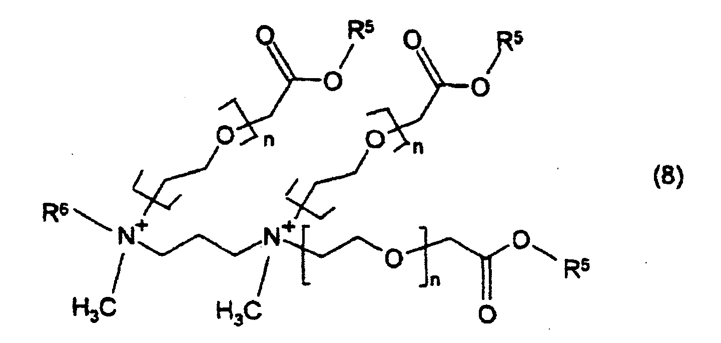

9. Composés de formule 1 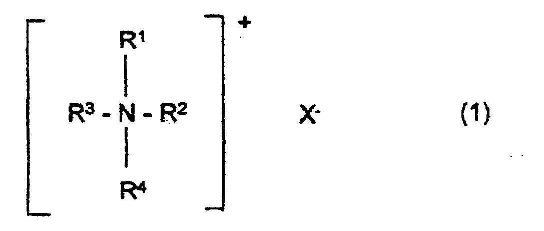 dans laquelle
R¹, R² représentent un radical de formule
-(A-O)ₙ-(C)-CO-O-R⁵ (3)
R³ un groupe alkyle en C₁ à C₃₀ ou alcényle en C₂ à C₃₀
R⁴ un radical organique, contenant le cas échéant des hétéroatomes, comportant de 1 à 100 atomes de carbone
R⁵ un groupe alkyle en C₁ à C₃₀ ou alcényle en C₂ à C₃₀
n un nombre de 1 à 20
A un groupe alkylène en C₂ à C₄
C un groupe alkylène en C₁ à C₆
X un anion.
